(19) Europäisches Patentamt
European Patent Office
Office européen des brevets

(11) **EP 4 364 566 A1**

(12) **EUROPEAN PATENT APPLICATION**

(43) Date of publication:
**08.05.2024 Bulletin 2024/19**

(21) Application number: **22205503.0**

(22) Date of filing: **04.11.2022**

(51) International Patent Classification (IPC):
*A01N 25/10* (2006.01)    *A01N 37/18* (2006.01)
*A61K 38/12* (2006.01)    *A01P 3/00* (2006.01)

(52) Cooperative Patent Classification (CPC):
**A01N 37/18; A01N 25/10; A61K 38/12**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **B. Braun Melsungen AG
34212 Melsungen (DE)**

(72) Inventors:
• **SCHWEBEL, Herve**
  **34212 Melsungen (DE)**
• **JUIF, Olivier**
  **34212 Melsungen (FR)**
• **ADAMO, Vincent**
  **34212 Melsungen (DE)**

(74) Representative: **Maiwald GmbH
Elisenhof
Elisenstraße 3
80335 München (DE)**

(54) **ANTI-FUNGAL COMPOSITION**

(57)    The present disclosure relates to an anti-fungal composition comprising caspofungin, or a pharmaceutically acceptable salt thereof and at least one bulking agent, which does not comprise a buffering agent; and to a sealed container comprising said anti-fungal composition, as well as to a method of manufacturing thereof.

## Description

### Field of the invention

The present invention relates to an anti-fungal composition comprising caspofungin, or a pharmaceutically acceptable salt thereof, and at least one bulking agent, as well as to a method of manufacturing of said anti-fungal composition, an anti-fungal composition prepared by such method, a sealed container comprising said anti-fungal composition, and an anti-fungal composition for use in the treatment or prevention of a fungal infection.

### Background

Caspofungin is a lipo-peptide anti-fungal drug from the class of echinocandins which is particularly effective in treating or preventing fungal infections caused by *Aspergillus* and *Candida* species. On the molecular level, caspofungin inhibits the fungal enzymatic (1→3)-β-D-glucan synthesis, which is an important component of the fungal cell walls. Upon inhibition of the (1→3)-β-D-glucan synthesis, the cell walls become defective and brittle and the fungus can no longer continue to grow. Since (1→3)-β-D-glucan does not occur naturally in the cell walls of mammals, caspofungin is unlikely to be harmful to the cells of an infected patient, such as a human patient.

For the high molecular weight of about 1,093.3 g/mol and its specific lipo-peptide structure, caspofungin is practically not bioavailable after oral administration. Therefore, caspofungin or its pharmaceutically acceptable salts are usually administered intravenously in the form of an aqueous formulations.

For that, caspofungin or a pharmaceutically acceptable salt thereof is typically commercialized as a lyophilized powder, which can be reconstituted by medical professionals before intravenous administration to the patient. Nevertheless, a significant challenge associated with the commercially available formulations of caspofungin is the inherent instability, in particular the high instability at ambient temperature. Upon storage of caspofungin lyophilized powders, degradation products of caspofungin acetate are rapidly formed through chemical decomposition reactions. Such degradation products include various hydrolysis products and dimers of caspofungin, as well as other non-characterized substances.

Although caspofungin and its pharmaceutically acceptable salts are marketed for several decades now, the mechanisms behind the formation of the impurities are not fully understood.

Therefore, several approaches have been described in an attempt to enhance the stability of caspofungin in a pharmaceutical composition. Such attempts focus on the selection of specific buffering systems, such as acetate buffers, and control of pH. Independently, it is essential to all known formulations of lyophilized caspofungin, such as the formulation Cancidas® marketed by Merck Sharp & Dohme, that said formulations must be stored at reduced temperatures in the range of from -70 °C to + 10 °C, such as in the refrigerator at 2 °C to 8 °C, to achieve an acceptable stability.

Cancidas® contains, besides the active ingredient caspofungin diacetate, acetic acid and sodium hydroxide as buffering agents, as well as sucrose and mannitol as bulking agents. Thereby, Cancidas® contains caspofungin diacetate and combined bulking agents sucrose and mannitol in a ratio of 1 : 1.3 (w/w).

### Summary

In view of the above, there is still need for formulations comprising caspofungin, or a pharmaceutically acceptable salt thereof, that can be stored at ambient or room temperature. Specifically, it is desirable for such formulation of caspofungin, or a pharmaceutically acceptable salt thereof to be stable at a temperatures of about 25 °C for a period of 2 years or more. At the same time, it is desirable that such stable formulation can be conveniently prepared, stored, reconstituted and administered.

The inventors of the present disclosure have surprisingly found that room temperature stable compositions of caspofungin, or a pharmaceutically acceptable salt thereof can be prepared without using a buffering agent, and by using caspofungin or a pharmaceutically acceptable salt thereof and at least one bulking agent in a specific weight ratio.

According to a first aspect, the present disclosure relates to an anti-fungal composition comprising caspofungin, or a pharmaceutically acceptable salt thereof, and at least one bulking agent, wherein the ratio between the amount of caspofungin, or a pharmaceutically acceptable salt thereof, and the amount of bulking agent(s) is at least about 1 : 2 (w/w), and wherein the anti-fungal composition does not comprise a buffering agent.

According to a second aspect, the present disclosure relates to a method of manufacturing an anti-fungal composition according to the first aspect of the present disclosure, the method comprising the steps of

    a) providing a first solution of at least one bulking agent in water;
    b) dissolving caspofungin or its pharmaceutically acceptable salt in said first solution;
    c) freezing the solution obtained in step b) at a temperature of from about -50 °C to about -40 °C for about 3 h to about 10 h to provide a frozen solution; and

d) freeze drying said frozen solution to provide the anti-fungal composition.

According to a third aspect, the present disclosure relates to an anti-fungal composition prepared by the method of the second aspect of the present disclosure.

According to a fourth aspect, the present disclosure relates to a sealed container comprising the anti-fungal composition according to the first aspect of the present disclosure or the third aspect of the present disclosure.

According to a fifth aspect, the present disclosure relates to an anti-fungal composition according to the first aspect of the present disclosure or the third aspect of the present disclosure for use in the treatment or prevention of a fungal infection.

**Detailed Description**

Anti-fungal composition

According to a first aspect, the present disclosure relates to an anti-fungal composition comprising caspofungin, or a pharmaceutically acceptable salt thereof, and at least one bulking agent, wherein the ratio between the amount of caspofungin, or a pharmaceutically acceptable salt thereof, and the amount of bulking agent(s) is at least about 1 : 2 (w/w), and wherein the anti-fungal composition does not comprise a buffering agent.

The inventors of the present disclosure have surprisingly found that anti-fungal compositions comprising caspofungin, or a pharmaceutically acceptable salt thereof, have excellent stability at room temperature and 60 % relative humidity, as well as under several stress conditions of higher temperature and/or particularly high relative humidity, when the anti-fungal composition does not comprise a buffering agent. Instead, the inventors have surprisingly found that the ratio between the amount of caspofungin, or a pharmaceutically acceptable salt thereof, is essential for the stability of caspofungin in an anti-fungal composition in the absence of a buffering agent.

It is understood that the anti-fungal composition according to the present disclosure may advantageously be stored in a sealed container, such as is a glass vial or a plastic vial.

Additionally, the anti-fungal compositions, e.g. in the form of a freeze dried cake or powder, i.e. in a lyophilized form, can be readily stored at room temperature for 24 months or longer without showing considerable decomposition of caspofungin. In other words, the anti-fungal compositions according to the present disclosure can be stored without using a fridge or freezer as shown in example 4.

*Caspofungin*

The anti-fungal composition comprises caspofungin of the present disclosure comprises caspofungin, or a pharmaceutically acceptable salt thereof.

According to a preferred embodiment of the present disclosure, the anti-fungal composition comprises caspofungin. According to another preferred embodiment of the present disclosure, the anti-fungal composition comprises a pharmaceutically acceptable salt of caspofungin. According to another preferred embodiment of the present disclosure, the anti-fungal composition comprises a caspofungin salt of acetic acid, citric acid, tartaric acid, propionic acid, succinic acid, oxalic acid, malic acid, maleic acid, lactic acid, or glutamic acid, preferably a caspofungin salt of acetic acid, more preferably caspofungin acetate or caspofungin diacetate.

According to a further preferred embodiment of the present disclosure, the anti-fungal composition comprises a caspofungin salt of acetic acid. According to a further preferred embodiment of the present disclosure, the anti-fungal composition comprises the acetate salt of caspofungin. According to a further preferred embodiment of the present disclosure, the anti-fungal composition comprises the diacetate salt of caspofungin. The latter is a particularly preferred salt of caspofungin.

According to another preferred embodiment of the present disclosure, the anti-fungal composition comprises a caspofungin salt of citric acid. According to another preferred embodiment of the present disclosure, the anti-fungal composition comprises a caspofungin salt of tartaric acid. According to another preferred embodiment of the present disclosure, the anti-fungal composition comprises a caspofungin salt of propionic acid. According to another preferred embodiment of the present disclosure, the anti-fungal composition comprises a caspofungin salt of succinic acid. According to another preferred embodiment of the present disclosure, the anti-fungal composition comprises a caspofungin salt of oxalic acid. According to another preferred embodiment of the present disclosure, the anti-fungal composition comprises a caspofungin salt of malic acid. According to another preferred embodiment of the present disclosure, the anti-fungal composition comprises a caspofungin salt of maleic acid. According to another preferred embodiment of the present disclosure, the anti-fungal composition comprises a caspofungin salt of lactic acid. According to another preferred embodiment of the present disclosure, the anti-fungal composition comprises a caspofungin salt of glutamic acid.

According to another preferred embodiment of the present disclosure, the anti-fungal composition comprises caspofungin, or a pharmaceutically acceptable salt thereof, in an amount of from about 8 $\mu$mol to about 125 $\mu$mol. According to another preferred embodiment of the present disclosure, the anti-fungal composition comprises caspofungin, or a pharmaceutically acceptable salt thereof in an amount of from about 20 $\mu$mol to about 85 $\mu$mol. According to another preferred embodiment of the present disclosure, the anti-fungal composition comprises caspofungin, or a pharmaceutically acceptable salt thereof in an amount of from about 40 $\mu$mol to about 65 $\mu$mol. According to another preferred embodiment of the present disclosure, the anti-fungal composition comprises caspofungin, or a pharmaceutically acceptable salt thereof in an amount of from about 60 $\mu$mol to about 70 $\mu$mol. According to another preferred embodiment of the present disclosure, the anti-fungal composition comprises caspofungin, or a pharmaceutically acceptable salt thereof in an amount of from about 63 $\mu$mol to about 65 $\mu$mol. According to another preferred embodiment of the present disclosure, the anti-fungal composition comprises caspofungin, or a pharmaceutically acceptable salt thereof in an amount of about 64 $\mu$mol. According to another preferred embodiment of the present disclosure, the anti-fungal composition comprises caspofungin, or a pharmaceutically acceptable salt thereof in an amount of from about 40 $\mu$mol to about 50 $\mu$mol. According to another preferred embodiment of the present disclosure, the anti-fungal composition comprises caspofungin, or a pharmaceutically acceptable salt thereof in an amount of from about 45 $\mu$mol to about 47 $\mu$mol. According to another preferred embodiment of the present disclosure, the anti-fungal composition comprises caspofungin, or a pharmaceutically acceptable salt thereof in an amount of about 46 $\mu$mol.

*Bulking agent*

The anti-fungal composition of the present disclosure comprises at least one bulking agent, such as 1, 2, 3, 4, 5, 6, 7, or 8 bulking agents.

According to a preferred embodiment of the present disclosure, the anti-fungal composition comprises one bulking agent. According to another preferred embodiment of the present disclosure, the anti-fungal composition comprises two or more bulking agents. According to another preferred embodiment of the present disclosure, the anti-fungal composition comprises two bulking agents. According to another preferred embodiment of the present disclosure, the anti-fungal composition comprises three bulking agents. According to another preferred embodiment of the present disclosure, the anti-fungal composition comprises four bulking agents.

According to another preferred embodiment of the present disclosure, the at least one bulking agent is selected from the group consisting of a monosaccharide, a sugar alcohol, a disaccharide, and a polysaccharide, preferably wherein the at least one bulking agent comprises a disaccharide or a polysaccharide, more preferably wherein the at least one bulking agent comprises a disaccharide, most preferably wherein the at least one bulking agent comprises sucrose.

According to another preferred embodiment of the present disclosure, the at least one bulking agent comprises a monosaccharide. According to another preferred embodiment of the present disclosure, the at least one bulking agent comprises a sugar alcohol. According to another preferred embodiment of the present disclosure, the at least one bulking agent comprises a disaccharide. According to another preferred embodiment of the present disclosure, the at least one bulking agent comprises a polysaccharide. According to another preferred embodiment of the present disclosure, the at least one bulking agent comprises sucrose. According to another preferred embodiment of the present disclosure, the at least one bulking agent comprises a dextran, preferably Dextran 40.

It has surprisingly been found that any of the above listed bulking agents, alone or in combination, are particularly useful in providing an anti-fungal composition with good stability properties as evidenced in examples 1 and 3.

According to another preferred embodiment of the present disclosure, the ratio between the amount of caspofungin, or a pharmaceutically acceptable salt thereof, and the amount of bulking agent(s) is at least 1 : 5 (w/w), preferably at least 1 : 7 (w/w), more preferably at least 1 : 8 (w/w), more preferably at least 1 : 9 (w/w), more preferably at least 1 : 10 (w/w), more preferably at least 1 : 11 (w/w), more preferably at least 1 : 12 (w/w), more preferably at least 1 : 13 (w/w), most preferably at least 1 : 14 (w/w).

According to another preferred embodiment of the present disclosure, the ratio between the amount of caspofungin, or a pharmaceutically acceptable salt thereof, and the amount of bulking agent(s) is more than 1 : 2 (w/w). According to another preferred embodiment of the present disclosure, the ratio between the amount of caspofungin, or a pharmaceutically acceptable salt thereof, and the amount of bulking agent(s) is at least 1 : 5 (w/w). According to another preferred embodiment of the present disclosure, the ratio between the amount of caspofungin, or a pharmaceutically acceptable salt thereof, and the amount of bulking agent(s) is at least 1 : 7 (w/w). According to another preferred embodiment of the present disclosure, the ratio between the amount of caspofungin, or a pharmaceutically acceptable salt thereof, and the amount of bulking agent(s) is at least 1 : 8 (w/w). According to another preferred embodiment of the present disclosure, the ratio between the amount of caspofungin, or a pharmaceutically acceptable salt thereof, and the amount of bulking agent(s) is at least 1 : 9 (w/w). According to another preferred embodiment of the present disclosure, the ratio between the amount of caspofungin, or a pharmaceutically acceptable salt thereof, and the amount of bulking agent(s) is at least 1 : 10 (w/w). According to another preferred embodiment of the present disclosure, the ratio between the amount of caspofungin, or a pharmaceutically acceptable salt thereof, and the amount of bulking agent(s) is at least 1 : 11 (w/w). According to another preferred embodiment of the present disclosure, the ratio between the amount of caspofungin, or a pharmaceutically acceptable salt thereof, and the amount of bulking agent(s) is at least 1 : 12 (w/w). According to another preferred embodiment of the present disclosure, the ratio between the amount of caspofungin, or a pharmaceutically acceptable salt thereof, and the amount of bulking agent(s) is at least 1 : 13 (w/w). According to another preferred embodiment of the present disclosure, the ratio between the amount of caspofungin, or a pharmaceutically acceptable salt thereof, and the amount of bulking agent(s) is at least 1 : 14 (w/w).

According to another preferred embodiment of the present disclosure, the ratio between the amount of caspofungin, or a pharmaceutically acceptable salt thereof, and the amount of bulking agent(s) is 1 : 40 (w/w) or less. According to another preferred embodiment of the present disclosure, the ratio between the amount of caspofungin, or a pharmaceutically acceptable salt thereof, and the amount of bulking agent(s) is 1 : 35 (w/w) or less. According to another preferred embodiment of the present disclosure, the ratio between the amount of caspofungin, or a pharmaceutically acceptable salt thereof, and the amount of bulking agent(s) is 1 : 33 (w/w) or less. According to another preferred embodiment of the present disclosure, the ratio between the amount of caspofungin, or a pharmaceutically acceptable salt thereof, and the amount of bulking agent(s) is 1 : 30 (w/w) or less. According to another preferred embodiment of the present disclosure, the ratio between the amount of caspofungin, or a pharmaceutically acceptable salt thereof, and the amount of bulking agent(s) is 1 : 28 (w/w) or less. According to another preferred embodiment of the present disclosure, the ratio between the amount of caspofungin, or a pharmaceutically acceptable salt thereof, and the amount of bulking agent(s) is 1 : 26 (w/w) or less. According to another preferred embodiment of the present disclosure, the ratio between the amount of caspofungin, or a pharmaceutically acceptable salt thereof, and the amount of bulking agent(s) is 1 : 25 (w/w) or less. According to another preferred embodiment of the present disclosure, the ratio between the amount of caspofungin, or a pharmaceutically acceptable salt thereof, and the amount of bulking agent(s) is 1 : 24 (w/w) or less. According to another preferred embodiment of the present disclosure, the ratio between the amount of caspofungin, or a pharmaceutically acceptable salt thereof, and the amount of bulking agent(s) is 1 : 23 (w/w) or less. According to another preferred embodiment of the present disclosure, the ratio between the amount of caspofungin, or a pharmaceutically acceptable salt thereof, and the amount of bulking agent(s) is 1 : 22 (w/w) or less.

According to another preferred embodiment of the present disclosure, the ratio between the amount of caspofungin, or a pharmaceutically acceptable salt thereof, and the amount of bulking agent(s) is in the range of from about 1 : 5 (w/w) to about 1 : 40 (w/w), preferably in the range of from about 1 : 7 (w/w) to about 1 : 35 (w/w), more preferably in the range of from about 1 : 8 (w/w) to about 1 : 33 (w/w), more preferably in the range of from about 1 : 9 (w/w) to about 1 : 30 (w/w), more preferably in the range of from about 1 : 10 (w/w) to about 1 : 28 (w/w), more preferably in the range of from about 1 : 11 (w/w) to about 1 : 26 (w/w), more preferably in the range of from about 1 : 12 (w/w) to about 1 : 25 (w/w), more preferably in the range of from about 1 : 13 (w/w) to about 1 : 24 (w/w), more preferably in the range of from about 1 : 14 (w/w) to about 1 : 23 (w/w), most preferably in the range of from about 1 : 14 (w/w) to about 1 : 22 (w/w).

According to another preferred embodiment of the present disclosure, the ratio between the amount of caspofungin, or a pharmaceutically acceptable salt thereof, and the amount of bulking agent(s) is in the range of from about 1 : 5 (w/w) to about 1 : 40 (w/w). According to another preferred embodiment of the present disclosure, the ratio between the amount of caspofungin, or a pharmaceutically acceptable salt thereof, and the amount of bulking agent(s) is in the range of from about 1 : 7 (w/w) to about 1 : 35 (w/w). According to another preferred embodiment of the present disclosure, the ratio between the amount of caspofungin, or a pharmaceutically acceptable salt thereof, and the amount of bulking agent(s) is in the range of from about 1 : 8 (w/w) to about 1 : 33 (w/w). According to another preferred embodiment of the present disclosure, the ratio between the amount of caspofungin, or a pharmaceutically acceptable salt thereof, and the amount of bulking agent(s) is in the range of from about 1 : 9 (w/w) to about 1 : 30 (w/w). According to another preferred embodiment of the present disclosure, the ratio between the amount of caspofungin, or a pharmaceutically acceptable salt thereof, and the amount of bulking agent(s) is in the range of from about 1 : 10 (w/w) to about 1 : 28 (w/w). According to another preferred embodiment of the present disclosure, the ratio between the amount of caspofungin, or a pharmaceutically acceptable salt thereof, and the amount of bulking agent(s) is in the range of from about 1 : 11 (w/w) to about 1 : 26 (w/w). According to another preferred embodiment of the present disclosure, the ratio between the amount of caspofungin, or a pharmaceutically acceptable salt thereof, and the amount of bulking agent(s) is in the range of from about 1 : 12 (w/w) to about 1 : 25 (w/w). According to another preferred embodiment of the present disclosure, the ratio between the amount of caspofungin, or its pharmaceutically acceptable salt, and the amount of bulking agent(s) is in the range of from about 1 : 13 (w/w) to about 1 : 24 (w/w). According to another preferred embodiment of the present disclosure, the ratio between the amount of caspofungin, or a pharmaceutically acceptable salt thereof, and the amount of bulking agent(s) is in the range of from about 1 : 14 (w/w) to about 1 : 23 (w/w). According to another preferred embodiment of the present disclosure, the ratio between the amount of caspofungin, or a pharmaceutically acceptable salt thereof, and the amount of bulking agent(s) is in the range of from about 1 : 14 (w/w) to about 1 : 22 (w/w).

The inventors of the present disclosure have surprisingly found that anti-fungal compositions having a specific ratio between caspofungin, or a pharmaceutically salt thereof, and the amount of bulking agent(s) show an increased stability of caspofungin in the anti-fungal composition as shown in examples 1 and 3. Without being bound by theory, it may be assumed that anti-fungal compositions having such specific ratio may be dried, e.g. freeze-dried, more conveniently and hence have a low content of water. Furthermore, it was surprisingly found that anti-fungal compositions having such specific ratio are less prone to taking up water from the atmosphere. In other words, the anti-fungal compositions having such specific ratio are less sensitive to humidity. Therefore, hydrolyzation reactions of caspofungin in the anti-fungal compositions are not very common, or are completely inhibited.

*Further Properties*

According to another preferred embodiment of the present disclosure, the anti-fungal composition is in the form of a lyophilized composition. According to a further preferred embodiment of the present disclosure, the anti-fungal composition is in the form of a lyophilized composition, wherein said lyophilized composition is suitable for reconstitution to form a liquid composition for parenteral administration.

It has been surprisingly found by the inventors of the present disclosure, that the anti-fungal compositions according to the first aspect of the present disclosure can be conveniently freeze dried and yield lyophilized compositions, such as cakes or powders, that can be readily reconstituted upon addition of water to form an injectable formulation of caspofungin, or a pharmaceutically acceptable salt thereof. Reconstitution does not involve excessive shaking and hence can be readily performed by a medical practitioner.

Additionally, the anti-fungal compositions in the form of a freeze dried cake or powder, i.e. in a lyophilized form, can be readily stored at room temperature for 24 months or longer without showing considerable decomposition of caspofungin. In other words, the anti-fungal compositions according to the present disclosure can be stored without using a fridge or freezer.

According to another preferred embodiment of the present disclosure, the anti-fungal composition has a density of about 0.5 g/mL or less. According to another preferred embodiment of the present disclosure, the anti-fungal composition has a density of about 0.3 g/mL or less. According to another preferred embodiment of the present disclosure, the anti-fungal composition has a density of about 0.2 g/mL or less. According to another preferred embodiment of the present disclosure, the anti-fungal composition has a density in the range of from about 0.1 to about 0.2 g/mL.

It is understood that the density of the anti-fungal composition according to the present disclosure as used herein refers to the density of the anti-fungal composition in a solid form, such as in the form of a lyophilized powder (see also further definition below).

It has been surprisingly found by the inventors of the present disclosure that the anti-fungal compositions according to the first aspect of the present disclosure having a specific density exhibit particularly low water content after freeze drying as shown in example 2. Without being bound by theory, it may be assumed that hydrolyzation reactions of caspofungin in the anti-fungal composition are thus effectively reduced or inhibited.

According to another preferred embodiment of the present disclosure, the anti-fungal composition has a water content of about 2 wt.-% or less based on the total weight of the anti-fungal composition. According to another preferred embodiment of the present disclosure, the anti-fungal composition has a water content of about 1.5 wt.-% or less based on the total weight of the anti-fungal composition. According to another preferred embodiment of the present disclosure, the anti-fungal composition has a water content of about 1 wt.-% or less based on the total weight of the anti-fungal composition.

It has been surprisingly found by the inventors of the present disclosure that the anti-fungal compositions according to the first aspect of the present disclosure having a specifically low water content exhibit excellent stability as shown in examples 1 to 4. Without being bound by theory, it may be assumed that hydrolyzation reactions of caspofungin in the anti-fungal composition are thus effectively reduced or inhibited. Therefore, the anti-fungal composition according to the present disclosure can be stored outside a freezer or a fridge, i.e. at ambient or even elevated temperatures for long time, such as 6 months, 12 months, 18 months, 24 months, or even longer without showing decomposition. In other words, the fungal compositions according to the first aspect of the present disclosure have high stability and long shelf life or shelf storage.

According to another preferred embodiment of the present disclosure, the anti-fungal composition has a shelf storage of at least 24 months. According to another preferred embodiment of the present disclosure, the anti-fungal composition comprises at least 95 % (n/n) after storage for 24 months at 25 °C and 60 % relative humidity based on the amount of caspofungin or pharmaceutically acceptable salt thereof before storage. According to another preferred embodiment of the present disclosure, the anti-fungal composition has a shelf storage of at least 24 months. According to another preferred embodiment of the present disclosure, the anti-fungal composition comprises at least 96 % (n/n) after storage for 24 months at 25 °C and 60 % relative humidity based on the amount of caspofungin or pharmaceutically acceptable salt thereof before storage. According to another preferred embodiment of the present disclosure, the anti-fungal composition has a shelf storage of at least 24 months. According to another preferred embodiment of the present disclosure, the anti-fungal composition comprises at least 97 % (n/n) after storage for 24 months at 25 °C and 60 % relative humidity based on the amount of caspofungin or pharmaceutically acceptable salt thereof before storage. According to another preferred embodiment of the present disclosure, the anti-fungal composition has a shelf storage of at least 24 months. According to another preferred embodiment of the present disclosure, the anti-fungal composition comprises at least 98 % (n/n) after storage for 24 months at 25 °C and 60 % relative humidity based on the amount of caspofungin or pharmaceutically acceptable salt thereof before storage.

According to another preferred embodiment of the present disclosure, the anti-fungal composition comprises 5 % or less of impurities after storage for 24 months at 25 °C and 60 % relative humidity. According to another preferred embodiment of the present disclosure, the anti-fungal composition comprises 4 % or less of impurities after storage for 24 months at 25 °C and 60 % relative humidity. According to another preferred embodiment of the present disclosure, the anti-fungal composition comprises 3 % or less of impurities after storage for 24 months at 25 °C and 60 % relative humidity. According to another preferred embodiment of the present disclosure, the anti-fungal composition comprises 2 % or less of impurities after storage for 24 months at 25 °C and 60 % relative humidity.

According to another preferred embodiment of the present disclosure, the anti-fungal composition comprises 5 % or less of impurities after storage for 8 months at 40 °C. According to another preferred embodiment of the present disclosure, the anti-fungal composition comprises 4 % or less of impurities after storage for 8 months at 40 °C. According to another preferred embodiment of the present disclosure, the anti-fungal composition comprises 3 % or less of impurities after storage for 8 months at 40 °C. According to another preferred embodiment of the present disclosure, the anti-fungal composition comprises 2 % or less of impurities after storage for 8 months at 40 °C.

According to another preferred embodiment of the present disclosure, the anti-fungal composition comprises 5 % or less of impurities after storage for 18 months at 30 °C. According to another preferred embodiment of the present disclosure, the anti-fungal composition comprises 4 % or less of impurities after storage for 18 months at 30 °C. According to another preferred embodiment of the present disclosure, the anti-fungal composition comprises 3 % or less of impurities after storage for 18 months at 30 °C. According to another preferred embodiment of the present disclosure, the anti-fungal composition comprises 2 % or less of impurities after storage for 18 months at 30 °C.

Method of manufacture

According to a second aspect, the present disclosure relates to a method of manufacturing an anti-fungal composition according to the first aspect and its preferred embodiments, comprising the steps of

a) providing a first solution of at least one bulking agent in water;
b) dissolving caspofungin or its pharmaceutically acceptable salt in said first solution;
c) freezing the solution obtained in step b) at a temperature of from about -60 °C to about -40 °C for about 3 h to about 10 h to provide a frozen solution; and
d) freeze drying said frozen solution to provide the anti-fungal composition.

It is understood that a buffering agent is not used in the method of manufacture, thereby preparing an anti-fungal composition not comprising a buffering agent.

According to a preferred embodiment of the present disclosure, the first solution comprises the at least one bulking agent in an amount of from about 2 wt.-% to about 25 wt.-% based on the total weight of the first solution. According to a preferred embodiment of the present disclosure, the first solution comprises the at least one bulking agent in an amount of from about 5 wt.-% to about 20 wt.-% based on the total weight of the first solution. According to a preferred embodiment of the present disclosure, the first solution comprises the at least one bulking agent in an amount of from about 7 wt.-% to about 15 wt.-% based on the total weight of the present disclosure. According to a preferred embodiment of the present disclosure, the first solution comprises the at least one bulking agent in an amount of from about 8 wt.-% to about 12 wt.-% based on the total weight of the first solution. According to a preferred embodiment of the present disclosure, the first solution comprises the at least one bulking agent in an amount of from about 9 wt.-% to about 11 wt.-% based on the total weight of the first solution. According to a preferred embodiment of the present disclosure, the first solution comprises the at least one bulking agent in an amount of about 10 wt.-% based on the total weight of the first solution.

The inventors of the present disclosure have surprisingly found that it is advantageous that the first solution comprises the at least one bulking agent in a specific concentration as shown in example 2. Such concentration allows for the formation of anti-fungal compositions having a low density and high porosity. This, in turn, allows for fast and energy efficient freeze drying and yields anti-fungal compositions having low water content and excellent stability.

According to a preferred embodiment of the present disclosure, the solution obtained in step b) of the method according to the present disclosure is cooled to a temperature in the range of about -60 °C to about -40 °C within a time of about 2 h to about 3 h in step c) to provide a frozen solution. In other words, the solution obtained in step b) of the method according to the present disclosure is cooled at a rate of about 0.3 °C/h to about 0.7 °C/h, preferably at a rate of about 0.3 °C/h to about 0.5 °C/h, more preferably at a rate of about 0.3 °C/h to about 0.4 °C/h in step c) to provide a frozen solution. It is understood that the solution obtained from step b) of the method according to the present disclosure is cooled at atmospheric pressure in step c) to provide a frozen solution.

It is understood that step c) may further comprise a period of holding at a temperature in the range of from about -60 °C to about -40 °C for a holding period of from about 2 h to about 24 h to provide a frozen solution. According to a preferred embodiment, step c) further comprises a period of holding at a temperature in the range of from about -60 °C to about -40 °C for a holding period of from about 2 h to about 24 h to provide a frozen solution. According to a preferred embodiment, step c) further comprises a period of holding at a temperature in the range of from about -60 °C to about -40 °C for a holding period of from about 5 h to about 15 h to provide a frozen solution. According to a further preferred embodiment, step c) further comprises a period of holding at a temperature in the range of from about -60 °C to about -40 °C for a holding period of from about 8 h to about 12 h to provide a frozen solution. According to a still further preferred embodiment, step c) further comprises a period of holding at a temperature in the range of from about -60 °C to about -40 °C for a holding period of about 9 h to provide a frozen solution.

According to another preferred embodiment of the present disclosure, freeze drying the frozen solution in step d) is carried out for about 96 h or less, preferably for about 72 h or less.

According to another preferred embodiment of the present disclosure, freeze drying the frozen solution in step d) comprises a primary drying and a subsequent secondary drying. In other words, the product obtained from primary drying is subjected to a secondary drying. Primary and secondary drying differ in terms of temperature and pressure.

According to another preferred embodiment of the present disclosure, the primary drying is carried out at a temperature in the range of from about -60 °C to about -10 °C at reduced pressure. According to another preferred embodiment of the present disclosure, the primary drying is carried out at a temperature in the range of from about -60 °C to about -10 °C and at pressure in the range of from about 50 $\mu$bar to about 300 $\mu$bar. According to another preferred embodiment of the present disclosure, the primary drying is carried out at a temperature in the range of from about -60 °C to about -10 °C and at pressure in the range of from about 100 $\mu$bar to about 200 $\mu$bar. According to another preferred embodiment of the present disclosure, the primary drying is carried out at a temperature in the range of from about -60 °C to about -10 °C and at pressure in the range of from about 130 $\mu$bar to about 170 $\mu$bar. According to another preferred embodiment of the present disclosure, the primary drying is carried out at a temperature in the range of from about -60 °C to about -10 °C and at pressure of about 150 $\mu$bar.

According to another preferred embodiment of the present disclosure, the primary drying is carried out for a total period of time in the range of from about 10 h to about 80 h. According to another preferred embodiment of the present disclosure, the primary drying is carried out for a total period of time in the range of from about 15 h to about 75 h. According to another preferred embodiment of the present disclosure, the primary drying is carried out for a total period of time in the range of from about 20 h to about 70 h. According to another preferred embodiment of the present disclosure, the primary drying is carried out for a total period of time in the range of from about 30 h to about 65 h. According to another preferred embodiment of the present disclosure, the primary drying is carried out for a total period of time in the range of from about 40 h to about 65 h. According to another preferred embodiment of the present disclosure, the primary drying is carried out for a total period of time in the range of from about 45 h to about 65 h. According to another preferred embodiment of the present disclosure, the primary drying is carried out for a total period of time in the range of from about 45 h to about 60 h. According to another preferred embodiment of the present disclosure, the primary drying is carried out for a total period of time in the range of from about 50 h to about 60 h. According to another preferred embodiment of the present disclosure, the primary drying is carried out for a total period of time of about 55 h.

It is understood that the temperature may change in the course of the primary drying, but will always be in the range of from -60 °C to -10 °C. In other words, the temperature may be allowed to rise in the course of primary drying: The step of primary drying is initiated at the temperature of step c) in the range of from about -60 °C to about -40 °C, preferably at a temperature of about -50 °C. Said temperature is also referred to as the first primary drying temperature. Then, the temperature is subsequently allowed to increase to a second primary drying temperature in the range of from about -30 °C to about -10 °C, preferably to a second primary drying temperature of about -20 °C.

According to another preferred embodiment of the present disclosure, the temperature of the primary drying is allowed to increase from the temperature of step c) to the second primary drying temperature for a first period of time of about 5 h or less, preferably for a first period of time of about 2.5 h.

According to another preferred embodiment of the present disclosure, the primary drying is carried out at the second primary drying temperature for a second period of time of about 77 h or less. According to another preferred embodiment of the present disclosure, the primary drying is carried out at the second primary drying temperature for a second period of time in the range of from about 8 h to about 75 h. According to another preferred embodiment of the present disclosure, the primary drying is carried out at the second primary drying temperature for a second period of time in the range of from about 18 h to about 68 h. According to another preferred embodiment of the present disclosure, the primary drying is carried out at the second primary drying temperature for a second period of time in the range of from about 28 h to about 63 h. According to another preferred embodiment of the present disclosure, the primary drying is carried out at the second primary drying temperature for a second period of time in the range of from about 38 h to about 63 h. According to another preferred embodiment of the present disclosure, the primary drying is carried out at the second primary drying temperature for a second period of time in the range of from about 42 h to about 63 h. According to another preferred embodiment of the present disclosure, the primary drying is carried out at the second primary drying temperature for a second period of time in the range of from about 42 h to about 58 h. According to another preferred embodiment of the present disclosure, the primary drying is carried out at the second primary drying temperature for a second period of time in the range of from about 48 h to about 58 h. According to another preferred embodiment of the present disclosure, the primary drying is carried out at the second primary drying temperature for a second period of time in the range of from about 52 h to about 57 h.

It is understood that the total period of time for which the primary drying is carried out is the sum of the first period of time, during which the temperature is allowed to increase to the second primary drying temperature, and the second period of time, during which the primary drying is carried out at the second primary drying temperature.

According to another preferred embodiment of the present disclosure, the secondary drying is carried out at a temperature in the range of from about -10 °C to about 60 °C at reduced pressure. According to another preferred embodiment of the present disclosure, the secondary drying is carried out at a temperature in the range of from about -10 °C to about 60 °C and at pressure in the range of from about 0.1 μbar to about 100 μbar. According to another preferred embodiment of the present disclosure, the secondary drying is carried out at a temperature in the range of from about -10 °C to about 60 °C and at pressure in the range of from about 1 μbar to about 80 μbar. According to another preferred embodiment of the present disclosure, the secondary drying is carried out at a temperature in the range of from about -10 °C to about 60 °C and at pressure in the range of from about 5 μbar to about 50 μbar. According to another preferred embodiment of the present disclosure, the secondary drying is carried out at a temperature in the range of from about -10 °C to about 60 °C and at pressure of in the range of from about 10 μbar to about 30 μbar.

According to another preferred embodiment of the present disclosure, the secondary drying is carried out for a total period of time in the range of from about 5 h to about 50 h. According to another preferred embodiment of the present disclosure, the secondary drying is carried out for a total period of time in the range of from about 10 h to about 45 h. According to another preferred embodiment of the present disclosure, the secondary drying is carried out for a total period of time in the range of from about 15 h to about 40 h. According to another preferred embodiment of the present disclosure, the secondary drying is carried out for a total period of time in the range of from about 20 h to about 40 h. According to another preferred embodiment of the present disclosure, the secondary drying is carried out for a total period of time in the range of from about 25 h to about 35 h According to another preferred embodiment of the present disclosure, the secondary drying is carried out for a total period of time of about 30 h.

It is understood that the temperature may change in the course of the secondary drying, but will always be in the range of from -10 °C to 60 °C. In other words, it may be allowed to rise in the course of secondary drying: The step of secondary drying is initiated at a first secondary drying temperature in the range of from about -10 °C to about 0 °C, preferably at a first secondary drying temperature of about -10 °C. Then, the temperature of secondary drying is subsequently increased to a second secondary drying temperature in the range of from about 20 °C to about 60 °C, preferably to a second secondary drying temperature of about 40 °C.

According to another preferred embodiment of the present disclosure, the temperature of the secondary drying is increased from the first secondary drying temperature to the second secondary drying temperature for a first period of time of about 10 h or less, preferably for a first period of time of about 5 h.

According to another preferred embodiment of the present disclosure, the secondary drying is carried out at the second secondary drying temperature for a second period of time of about 45 h or less. According to another preferred embodiment of the present disclosure, the secondary drying is carried out at the second secondary drying temperature for a second period of time in the range of from about 5 h to about 40 h. According to another preferred embodiment of the present disclosure, the secondary drying is carried out at the second secondary drying temperature for a second period of time in the range of from about 40 h to about 35 h. According to another preferred embodiment of the present disclosure, the secondary drying is carried out at the second secondary drying temperature for a second period of time in the range of from about 15 h to about 35 h. According to another preferred embodiment of the present disclosure, the secondary drying is carried out at the second secondary drying temperature for a second period of time in the range of from about 20 h to about 30 h. According to another preferred embodiment of the present disclosure, the secondary drying is carried out at the second secondary drying temperature for a second period of time in the range of from about 23 h to about 27 h.

It is understood that the total period of time for which the secondary drying is carried out is the sum of the first period of time, during which the temperature is increased to the second secondary drying temperature, and the second period of time, during which the secondary drying is carried out at the second secondary drying temperature.

Anti-fungal composition prepared by method

According to a third aspect, the present disclosure relates to an anti-fungal composition prepared by the method of the second aspect of the present disclosure.

Sealed container

According to a fourth aspect, the present disclosure relates to a sealed container comprising the anti-fungal composition according to the first aspect of the present disclosure or the third aspect of the present disclosure.

According to a preferred embodiment of the present disclosure, the sealed container is a glass vial.

According to another preferred embodiment of the present disclosure, the sealed container is a plastic vial. According to another preferred embodiment of the present disclosure, the sealed container is a plastic vial comprises cyclo-olefin polymers (COP). According to another preferred embodiment of the present disclosure, the sealed container is a plastic vial comprises cyclo-olefin copolymers (COC).

Medical use

According to a fifth aspect, the present disclosure relates to an anti-fungal composition according to the first aspect of the present disclosure or the third aspect of the present disclosure for use in the treatment or prevention of a fungal infection.

Definitions and general embodiments

As used herein, the term "buffering agent" relates to a weak acid and its conjugate base. Buffering agents are usually added to water or an aqueous solution to form buffer solutions and are responsible for the buffering seen in these solutions. These agents may be added to substances that are to be placed into acidic or basic conditions in order to stabilize the substance. For example, buffered caspofungin has a buffering agent, such as acetic acid and an acetate, that will maintain the pH of the caspofungin formulation upon addition of acid or base. In other words, a buffering agent as used herein is a molecule or atom that maintains the pH of a composition at a specific value or in a specific range.

Non-limiting examples of buffering agents are formate buffer (formic acid/formate anion), acetate buffer (acetic acid/acetate anion), citrate buffer (citric acid/citrate anion), ammonium buffer ($NH_4^+/NH_3$), phosphate buffer ($H_3PO_4/H_2PO_4^-/HPO_4^{2-}/PO_4^{3-}$), fumarate buffer (fumaric acid/fumarate anion), carbonate buffer ($H_2CO_3/HCO_3^-/CO_3^{2-}$), sulfite buffer ($HSO_3^-/SO_3^{2-}$), maleate buffer (maleic acid/maleate anion), malonate buffer (malonic acid/malonate anion), HEPES buffer (2-[4-(2-Hydoxyethyl)piperazin-1-yl]ethane-1-sulfonic acid/anion thereof), MES buffer (2-(N-morpholino)ethanesulfonic acid/anion thereof), TRIS buffer (Tris(hydroxymethyl)aminommethan/cation thereof), propionate buffer (propionic acid/propionate anion), succinate buffer (succinic acid/succinate anion), glycolate buffer (glycolic acid/glycolate anion), gluconate buffer (gluconic acid/gluconate anion), lactate buffer (lactic acid/lactate anion), tartrate buffer (tartaric acid/tartrate), glutamate buffer (glutamic acid/glutamate anion), barbitone buffer (HCl/barbitone anion), barbiturate buffer (barbitone/barbitone anion), borax buffer ($Na_2B_4O_7$/NaOH), boric acid buffer ($H_3BO_3/Na_2B_4O_7$), glycine buffer (HCl/glycine or glycine/NaOH), and mixtures thereof.

As used herein, a "weak acid" is a substance that partially dissociates when it is dissolved water. In solution there is an equilibrium between the acid "HA" and the products of dissociation $H^+$ and $A^-$. As such, a weak acid has a $pK_a$ at 25 °C in the range of from 3 to 10. Non-limiting examples of weak acids are formic acid, acetic acid, citric acid, $NH4^+$, $H_2PO_4^-$, fumaric acid, $HCO_3^-$, $H_2CO_3$, $HSO_3^-$, maleic acid, malonic acid, HEPES, MES, TRIS, propionic acid, succinic acid, glycolic acid, gluconic acid, lactic acid, tartaric acid, glutamic acid, alkyl sulfonic acid, arylsulfonic acid, and the like.

As used herein, an anti-fungal composition does not comprise a buffering substance if the molar amount of caspofungin, or a pharmaceutically acceptable salt thereof is equal to or higher than the molar amount of weak acid. In other words, an anti-fungal composition does not comprise a buffering substance when the following equation is fulfilled:

*n(caspofungin or pharmaceutically acceptable salt of caspofungin) ≥ 2n(weak acid)*

Therefore, it is understood that the pharmaceutically acceptable anion of the pharmaceutically acceptable salt of caspofungin, and the conjugate acid of the pharmaceutically acceptable anion of the pharmaceutically acceptable salt of caspofungin is not a buffering agent in the sense of the present disclosure.

In other words, the anti-fungal composition according to the present disclosure does not contain anions of a weak acid apart from the anions of the pharmaceutically acceptable salt of caspofungin. For example, when the anti-fungal composition comprises caspofungin diacetate, the anti-fungal composition does not comprise further acetic acid or acetate anions thereby exceeding the molar amount of acetic acid/acetate stemming from the caspofungin diacetate.

As used herein, the term "caspofungin" relates to the free base of caspofungin, which is also known as (4R,5S)-

5-[(2-aminoethyl)amino]-N$^2$-(10,12-dimethyltetradecanoyl)-4-hydroxy-L-ornithyl-L-threonyl-trans-4-hydroxy-L-prolyl-(S)-4-hydroxy-4-(*p*-hydroxyphenyl)-L-threonyl-*threo*-3-hydroxy-L-ornithyl-*trans*-3-hydroxy-L-proline cyclic (6→1)-peptide, or 1-[(4*R*,5*S*)-5-[(2-Aminoethyl)amino]-N$^2$-(10,12-dimethyl-1-oxotetradecyl)-4-hydroxy-L-ornithine]-5-[(3*R*)-3-hydroxy-L-ornithine] pneumocandin B$_0$, or CAS number 162808-62-0. Caspofungin has the molecular formula C$_{52}$H$_{88}$N$_{10}$O$_{15}$ and a molecular weight of 1093.31 g/mol.

As used herein, the term "pharmaceutically acceptable salt of caspofungin" refers to a salt of caspofungin that retain the desired biological activity of the above-identified compounds and include pharmaceutically acceptable acid addition salts. Suitable pharmaceutically acceptable acid addition salts of caspofungin may be prepared from an inorganic acid or from an organic acid. Examples of such inorganic acids are hydrochloric, sulfuric, and phosphoric acid. Appropriate organic acids may be selected from aliphatic, cycloaliphatic, aromatic, heterocyclic carboxylic and sulfonic classes of organic acids, examples of which are formic, acetic, propionic, succinic, glycolic, gluconic, lactic, malic, tartaric, citric, fumaric, maleic, alkyl sulfonic, and arylsulfonic acid.

As used herein, the "diacetate salt of caspofungin" may also be referred to as caspofungin diacetate. Caspofungin diacetate is known under CAS number 179463-17-3 and has the molecular formula C$_{52}$H$_{88}$N$_{10}$O$_{15}$·2 C$_2$H$_4$O$_2$ and a molecular weight of 1213.42 g/mol.

As used herein, the term "monosaccharide" also called simple sugars, are the simplest forms of sugar and the most basic units (monomers) from which all carbohydrates are built. Hence, a monosaccharide cannot be decomposed into simpler sugars by hydrolysis. Non-limiting examples of monosaccharides are glucose, fructose, galactose, ribose and deoxyribose.

As used herein, the term "sugar alcohol" refers to an organic compound, typically derived from sugars or monosaccharides, containing one hydroxyl group (-OH) attached to each carbon atom. Non-limiting examples of sugar alcohols are sorbitol, mannitol, xylitol, arabitol, threitol, erythritol, ribitol, galactitol, fucitol, iditol, inositol, volemitol, isomalt, maltitol, and lactitol.

As used herein, the term "disaccharide" refers to an organic compound formed by glycosidic linkage of two monosaccharides. Non-limiting examples of disaccharides are sucrose, lactose, maltose, trehalose, cellobiose, chitobiose, kojibiose, nigerose, isomaltose, α ,β-trehaiose, β,β-trehalose, sophorose, laminaribiose, gentiobiose, trehalulose, turanose, maltulose, leucrose, isomaltulose, mannobiose, melibiose, melibiulose, rutinose, rutinulose, and xylobiose.

As used herein, the term "polysaccharide" refers to an organic compound formed by glycosidic linkage of more than two monosaccharides, such as of from 5 to 3,000 monosaccharides. Non-limiting examples of polysaccharides are starch, such as potato starch, rice starch, wheat starch, and maize starch; glycogen, galactogen, inulin, dextrans, arabinoxylans, celluloses, chitins, and pectins.

As used herein, the term "Dextran" refers to an organic molecule form the group of polysaccharides which is derived from the condensation of glucose; the main chain consists of α-1,6 glycosidic linkages between glucose monomers, with branches from α-1,3 linkages. In other words, dextranes are branched poly-α-d-glucosides having glycosidic bonds, predominantly C-1→C-6. Dextran chains are of varying lengths from 3 to 2000 kDa.

As used herein, the term "amount of bulking agent(s)" refers to the sum of the respective amounts of each of the at least one bulking agent. In other words, the amount of bulking agent(s) refers to the amount of all bulking agents comprised in the anti-fungal composition according to the present disclosure taken together. For example, if the anti-fungal composition according to the present disclosure comprises one bulking agent, the amount of bulking agent(s) is the amount of said one bulking agent. If the anti-fungal composition according to the present disclosure comprises two bulking agents, i.e. a first bulking agent and a second bulking agent, the amount of bulking agent(s) is the amount of said first bulking agent plus the amount of said second bulking agent.

As used herein, the term "parenteral administration" refers to a route of administration that is not enteral, such as subcutaneous administration or intravenous administration. According to a preferred embodiment of the present application, parenteral administration is intravenous administration.

As used herein, the "water content" of the anti-fungal composition is determined by coulometric Karl Fischer titration. For determining the water content, the coulometric Karl Fischer titrator System Metrohm 851 Titrando equipped with Double Pt wire electrode and Generator electrode without diaphragm. A defined amount of the anti-fungal composition is reconstituted with a defined volume of dry methanol. Then, the mixture is injected into the reaction vessel comprising Hydranal Coulomat AG reagent. Considering a polarizing current of 10 μA, the end point titration is determined for a 50 mV voltage with pre-defined requirements on initial and final signal drift. The water quantity in the composition is calculated by deducing the contribution of dry methanol diluent from the total water quantity determined in the titrated amount.

As used herein, the term "density" refers to the density of the anti-fungal composition in a solid form, such as in the form of a lyophilized powder. As used herein, the density of the anti-fungal composition according to the present disclosure is determined by weighing the amount of anti-fungal composition and determining the volume of said anti-fungal composition, which is calculated from the base area of a container comprising the anti-fungal composition and the height of the anti-fungal composition in said container. Methods for determining the base area and the height of an anti-fungal composition h (anti-fungal composition) are known to the skilled person. For example, when the container has the shape of a cylinder, which is common in the art, the density of the anti-fungal composition can be calculated according to the following formula:

$$density\ (anti-fungal\ composition) = \frac{weight\ (anti-fungal\ composition)}{\pi \cdot \left(\frac{d}{2}\right)^2 \cdot h(anti-fungal\ composition)}$$

wherein d is the diameter of the container.

As used herein, the term "shelf storage" means that the anti-fungal composition according to the present disclosure is stored at room temperature and room humidity. It is an object of the present disclosure that the anti-fungal composition remains stable during shelf storage. In other words, the amount of caspofungin comprised in the anti-fungal composition does not decrease during shelf storage. At the same time, the amount of impurities does not increase.

As used herein, the term "shelf life" relates to the time after preparation of an anti-fungal composition during which the anti-fungal composition has an acceptable purity when stored at room temperature and room humidity. Acceptable purity means that the anti-fungal composition at a specific time point, such as the shelf life, comprises 95 % (n/n), preferably 96 % (n/n), more preferably 97 % (n/n), most preferably 98 % (n/n) of caspofungin, or a pharmaceutically acceptable salt thereof based on the amount of caspofungin, or a pharmaceutically acceptable salt thereof directly after preparation of the anti-fungal composition.

As used herein, the term "room temperature" means 25 °C. As used herein, the term "room humidity" refers to 60 % relative humidity.

As used herein, the term "stable" means that the anti-fungal composition comprises at least 95 % (n/n), preferably at least 96 % (n/n), more preferably at least 97 % (n/n), most preferably at least 98 % (n/n) of caspofungin or its pharmaceutically acceptable salt after storage for 24 months at 25 °C and 60 % relative humidity based on the amount of caspofungin or pharmaceutically acceptable salt thereof before storage. In other words, the anti-fungal composition comprises 5 % or less of impurities, preferably 4 % or less of impurities, more preferably 3 % or less of impurities, most preferably 2 % of impurities after storage for 24 months at 25 °C and 60 % relative humidity based on the amount of caspofungin or pharmaceutically acceptable salt thereof before storage.

As used herein, the amount of caspofungin or a pharmaceutically acceptable salt thereof and the amount of impurities is determined by assay (I), or assay (II), or assay (III).

As used herein, the term "before storage" refers to the time point of fresh preparation of the anti-fungal composition. In other words, an anti-fungal composition according to the present disclosure is directly prepared and comprises caspofungin

Where the term "comprising" is used in the present description and claims, it does not exclude other elements. For the purposes of the present disclosure, the term "consisting of" is considered to be a preferred embodiment of the term "comprising". If a group is defined to comprise at least a certain number of embodiments herein, this is also to be understood to disclose a group, which preferably consists only of these embodiments. Furthermore, if a composition is defined using the term "comprising", it may additionally comprise other elements not explicitly listed, however, not further amounts of an element listed. As such, if, e.g., an anti-fungal composition comprises caspofungin, or a pharmaceutically acceptable salt thereof in an amount of 100 μmol, said anti-fungal-composition may comprise elements other than caspofungin, or a pharmaceutically acceptable salt thereof, however, not additional amounts of caspofungin, or a pharmaceutically acceptable salt thereof, thereby exceeding the amount of 100 μmol.

As used herein, the terms "impurity" or "impurities" refers to the total amount of impurities, unless specified otherwise. In other words, the terms "impurity" or "impurities" refer to the sum of the amounts of each individual impurity. It is understood that an impurity is any compound that is derived from caspofungin or a pharmaceutically acceptable salt thereof, such as through degradation reactions including oxidation and hydrolysis.

The term "about" in conjunction with a numerical value refers to normal deviations of said numerical value. It is to be understood that the term "about" can mean a deviation of ± 10 %, preferably ± 5 %, more preferably ± 2.5 % of said numeric value as indicated.

Assay (I)

For the determination of the chromatographic purity of caspofungin or a pharmaceutically acceptable salt thereof and the amount of impurities, a 18 minute reversed-phase liquid chromatography method is used. The analysis of 5 μL injected sample is performed on a HPLC Agilent 1200 system using a Waters Cortecs C18 column (150×4.6 mm, 2.7 μm, cat. n°# 186007408) maintained at 40 °C and at a flow rate of 0.50 mL/min. Elution conditions involve a gradient of binary mobile phases. Mobile phases consist of Solvent A (Ammonium acetate buffer adjusted at pH 3.45) and solvent B (acetonitrile/acetic acid; 99/1 (v/v)) with the following gradient elution program: 0 to 3.0 min solvent A from 75 to 50%, solvent B from 25 to 50 %; then holding until 5.0 min, 5.0 to 10.0 min solvent A from 50 to 2%, solvent B from 50 to 98 %; then holding until 13.0 min; 13.0 to 13.5 min solvent A from 2 to 75 %, solvent B from 98 % to 25 %, then holding until 18.0 min.

Caspofungin and its degradation products or impurities are detected by UV detection at 275 nm and the purity values in Area% are reported from the normalized area versus the caspofungin peak.

Assay (II)

For the determination of the amount of caspofungin or a pharmaceutically acceptable salt thereof and the amount of impurities, a 32 minute reversed-phase liquid chromatography method is used. For the assay, the anti-fungal composition is reconstituted to yield a solution containing a theoretical amount of 1.83 $\mu$mol/mL of caspofungin or a pharmaceutically acceptable salt thereof (corresponding to 2 mg/mL of caspofungin free base). The analysis of 4 $\mu$L injected sample is performed on a UPLC Waters Acquity H-Class system using a Waters Acquity CSH C18 column (150x3.0 mm, 1.7 $\mu$m, cat. n°#186005302) maintained at 45 °C and at a flow rate of 0.60 mL/min. Elution conditions involve a gradient of binary mobile phases. Mobile phases consist of Solvent A (70 mM phosphate buffer at pH 6.4/water/methanol, 6/1/3 (v/v)) and solvent B (acetonitrile/70 mM phosphate buffer pH 6.4/water; 14/6/1 (v/v)) with the following gradient elution program: 0 to 1 min 60 % solvent A and 40 % solvent B; 1 to 18 min solvent A from 60 to 20 %, solvent B from 40 to 80 %; 18 to 20 min solvent A from 80 to 100 %, solvent B from 20 % to 0 %, then holding until 5 min.

Caspofungin and its degradation products or impurities are detected by UV detection at 225 nm for caspofungin and 210 nm for impurities and the respective peak areas are calculated.

The amount of caspofungin and impurities in %, such as % (w/w), is performed by external calibration using a working standard of caspofungin whose purity assay was determined at each time point by a mass balance approach (100% - sum of impurities). For the quantification of impurities, the same response factor as for caspofungin is used.

Assay (III)

For the determination of the amount of caspofungin or a pharmaceutically acceptable salt thereof and the amount of impurities, a 50 minute reversed-phase liquid chromatography method is used. For the assay, the anti-fungal composition is reconstituted to yield a solution containing a theoretical amount of 1.83 $\mu$mol/mL of caspofungin or a pharmaceutically acceptable salt thereof (corresponding to 2 mg/mL of caspofungin free base) was prepared. The analysis of 2 $\mu$L (for assay) or 35 $\mu$L(for impurities) injected sample is performed on a HPLC Agilent 1200 system using a Waters XSelect CSH C18 column (100x4.6 mm, 2.5 $\mu$m, cat. n°#186006111) maintained at 45 °C and at a flow rate of 1.2 mL/min. Elution conditions involve a gradient of binary mobile phases. Mobile phases consist of Solvent A (50 mM phosphate buffer adjusted at pH 7.0/acetonitrile, 9/1 (v/v)) and solvent B (acetonitrile) with the following gradient elution program: 0 to 10 min solvent A from 83 % to 72%; solvent B from 17 to 28%, then holding until 20 min; 20 to 40 min solvent A from 72 to 33.5 %, solvent B from 28 to 66.5 %; 40 to 45 min solvent A from 33.5 to 83 %, solvent B from 66.5 to 17 %, then holding until 50 min.

Caspofungin and its degradation products or impurities are detected by UV detection at 225 nm for caspofungin and 210 nm for impurities and the respective peak areas are calculated.

The amount of caspofungin and impurities in %, such as % (w/w), is performed by external calibration using a working standard of caspofungin whose purity assay was determined by a mass balance approach (100% - sum of impurities).

**Examples**

Example 1

To investigate the effect of the type of bulking agents on the stability of caspofungin in a pharmaceutical compositions, the formulations as shown in Table 1 were prepared. The bulking agent was dissolved in water, and caspofungin was added. The solutions were frozen and freeze dried to provide a pharmaceutical composition. After freeze drying, the water content was measured by coulometric Karl Fischer titration at the beginning ($T_0$), and after the respective storage times.

After storage of the compositions at 25 C at 60 % relative humidity (rH) and under stress conditions at 40 °C at 75 % relative humidity (rH), the purity of the compositions was determined according to Assay (I).

Table 1 Overview on compositions and storage stability.

| Bulking agent | Ratio caspofungin / bulking agent (w/w) | To | Storage conditions 25 °C/ 60 % rH | | | Stress conditions 40 °C/75 % rH | |
|---|---|---|---|---|---|---|---|
| | | | 3-weeks | | 3-months | 3-weeks | 3-months |
| | | Water Content [wt.-%] | pH | Purity [A%] | Purity [A%] | Purity [A%] | Purity [A%] |
| Sucrose | 1 / 22 | 0.3 | 7.2 | 98.9 | 99.1 | 98.4 | 96.7 |
| | 1 / 2 | 1.0 | 7.4 | 97.5 | 98.8 | 95.8 | 45.0 |
| HES 130 | 1 / 22 | 0.3 | 8.4 | 98.4 | 96.9 | 92.3 | 87.5 |
| | 1 / 2 | 1.0 | 8.5 | 95.8 | 93.6 | 87.0 | 72.2 |
| Dextran 40 | 1 / 22 | 0.2 | 7.0 | 90.5 | - | 79.3 | - |
| | 1 / 2 | 1.1 | 7.0 | 90.0 | - | 73.9 | - |
| - means value not determined | | | | | | | |

The inventors have surprisingly found that the formulations with higher amount of excipient compared to caspofungin (in a ratio of 1 / 22 (w/w)) are more stable than compositions with lower excess of bulking agent (in a ratio of 1 / 2). Said effect can be observed for all bulking agents; especially under stress conditions at 40 °C and 75 % relative humidity (rH), the compositions comprising a large excess amount of bulking agents showed considerably slower degradation of caspofungin, i.e. they are more stable.

Furthermore, it was found that the lyophilized powders having a ratio of 1 / 2 caspofungin / bulking agent were less dry.

Example 2

To further evaluate the effect of the cake density, anti-fungal compositions were manufactured with two different weight ratios of caspofungin acetate to bulking agent, that are 1 : 14 (w/w) and 1 : 23 (w/w), prepared according to the method of example 1. Secondary drying was performed at 40 °C.

To investigate two different densities of the anti-fungal compositions, the vials were filled either with 1.0 ml of 5 % (w/v) sucrose solution (low density) or 0.2 ml of 25 % (w/v) sucrose solution (high density) before the lyophilization process. The initial water content was determined at the beginning ($T_0$). The samples were stored for 1 month (1M), 3 months (3M), or 8 months (8M).

Stability studies at low relative humidity conditions were performed to evaluate potential impact of environmental humidity conditions during storage. Purity was determined according to Assay (I).

Table 2 Overview on compositions and storage stability.

| Ratio caspofungin / bulking agent (w/w) | Property | To | Storage conditions 25°C | | | | Stress conditions 40°C | | |
|---|---|---|---|---|---|---|---|---|---|
| | | | <20 % rH | | | 60% rH | <20 % rH | | |
| | | | 1M | 3M | 8M | 8M | 1M | 3M | 8M |
| 1/14 (Low density) | Purity [A%] | - | 99.4 | 99.1 | 98.9 | 97.0 | 98.5 | 97.8 | 96.9 |
| | Water [%] | 0.4 | - | 0.7 | 0.8 | 0.9 | - | 0.9 | 0.9 |
| | pH | - | 7.3 | 7.3 | 7.3 | - | 7.3 | 7.3 | 7.3 |
| 1/23 (Low density) | Purity [A%] | - | 99.3 | 99.1 | 99.0 | - | 98.6 | 97.9 | 97.4 |
| | Water [%] | 0.5 | - | 0.8 | 0.8 | - | - | 0.9 | 1.0 |
| | pH | - | 7.4 | 7.3 | 7.3 | - | 7.3 | 7.3 | 7.2 |

(continued)

| Ratio caspofungin / bulking agent (w/w) | Property | To | Storage conditions 25°C | | | | Stress conditions 40°C | | |
|---|---|---|---|---|---|---|---|---|---|
| | | | <20 % rH | | | 60% rH | <20 % rH | | |
| | | | 1M | 3M | 8M | 8M | 1M | 3M | 8M |
| 1/14 (High density) | Purity [A%] | - | 98.8 | - | - | - | 97.6 | 96.4 | 52.9 |
| | Water [%] | 2.7 | - | - | - | - | - | - | 3.3 |
| | pH | - | 7.4 | - | - | - | 7.2 | 7.2 | 6.1 |
| 1/23 (High density) | Purity [A%] | - | 98.4 | - | - | - | 98.1 | 97.1 | 84.4 |
| | Water [%] | 2.3 | - | - | - | - | - | - | - |
| | pH | - | 7.1 | - | - | - | 7.1 | 7.1 | 6.8 |
| - means value not determined | | | | | | | | | |

It was demonstrated that compact freeze-dried samples resulting from a concentrated bulking agent solution before lyophilization led to a poor drying and high water content of the anti-fungal compositions after freeze drying. After 8-month storage at 40 °C in the compositions with the high density, the purity dropped drastically, and a pH decrease was observed.

On the other hand, the anti-fungal compositions with a low density (and low initial water content after drying), showed better chemical stability with very limited pH decrease and water content increase over the 8-month storage time. For said anti-fungal compositions, the increase of the weight ratio between caspofungin or pharmaceutically acceptably salt thereof and bulking agent from 1/14 to 1/23 (w/w) did not affect the chemical stability of caspofungin in the formulations.

Example 3

Anti-fungal compositions with weight ratios of caspofungin to bulking agent were prepared according to the method of Example 2, wherein the concentration of bulking agent in solution before freeze drying did not exceed 10 % (w/v) to prepare anti-fungal compositions of low density (table 3). The initial water content and the purity were determined at the beginning ($T_0$). The samples were stored for 4 weeks, or 6 weeks, respectively.

For Example 3, a raw material of caspofungin with already high impurity levels at $T_0$ (total impurities $\approx$ 2.2%) was used. Therefore, the purity results are not directly comparable to the results of Examples 1 and 2. Rather, the values given in column *"Formed Impurities"* represents the impurity increase, i.e. the amount of impurities formed during storage.

Stability studies at high relative humidity conditions were performed to evaluate potential impact of environmental humidity conditions during storage. Assay and Impurities were determined according to Assay (III).

**Table 3** Overview on compositions and storage stability.

| Bulking agent | Ratio Caspofungin / bulking agent (w/w) | $T_0$ | | | | Stress conditions (40 °C and 75 % rH) | | |
|---|---|---|---|---|---|---|---|---|
| | | pH | Assay [%] | Water [%] | Formed Impurities [%] | Assay [%] | Water [%] | Formed Impurities [%] |
| Sucrose | 1/2 | 6.9 | 98.1 | 0.1 | 0.75 | 92.1* | 0.5 | 2.0* |
| | 1/9 | 5.1 | 98.1 | 1.0 | 1.1 | 89.9* | 1.2 | 2.0* |
| | 1/9 | 6.7 | 97.3 | 1.0 | 0.64 | 91.9* | 0.8 | 1.0* |
| HES 130 | 1/2 | 7.0 | 89.6 | 0.2 | 4.9 | 56.0# | - | 8.8# |
| | 1/9 | 6.6 | 89.3 | 0.1 | 8.0 | 59.0# | - | 13.0# |

(continued)

| Bulking agent | Ratio Caspofungin / bulking agent (w/w) | T₀ | | | | Stress conditions (40 °C and 75 % rH) | | |
|---|---|---|---|---|---|---|---|---|
| | | pH | Assay [%] | Water [%] | Formed Impurities [%] | Assay [%] | Water [%] | Formed Impurities [%] |
| Dextran 40 | 1/2 | 6.8 | 90.0 | 0.5 | 4.3 | 59.4# | - | 6.3# |
| | 1/9 | 6.6 | 89.2 | 0.2 | 6.1 | 62.1# | - | 7.2# |
| * 4 weeks of storage<br># 6 weeks of storage<br>- means value not determined | | | | | | | | |

Example 4

A 24-month stability study was performed. For comparative purposes, a market product obtained from Fresenius Kabi (caspofungin acetate, 50 mg as free base) formulated with arginine was used, to compare the results in terms of water content, assay and impurity level.

In a 20R glass vial, 7.5 mL of 10% sucrose solution was lyophilized to achieve a high porosity anti-fungal composition with ratio of caspofungin / bulking agent of 1 /15 (w/w). The following precautions were taken to keep the water content as low as possible and therefore to limit the degradation of caspofungin: low temperature for bulk preparation, primary drying at -20 °C and secondary drying at 40 °C; preliminary drying of the bromobutyl Lyo stoppers at 110 °C, to prevent moisture release from the stoppers. The vials were stored at 25 °C and 60 % relative humidity.

The amount of caspofungin and impurities in the anti-fungal compositions were determined according to Assay (II). Water content was determined by coulometric Karl Fischer titration.

**Table 4** Overview on compositions and storage stability at 25 °C and 60 % relative humidity.

| | Anti-fungal composition | | | | | Fresenius Kabi composition |
|---|---|---|---|---|---|---|
| Time [months] | 0 | 6 | 12 | 18 | 24 | At end of shelf life |
| Water content [%] | 0.41* | - | - | 0.44* | 0.47† | 0.8† |
| pH | 6.7* | 6.7# | 6.7# | 6.7# | 6.6† | 6.7# |
| Assay [%] | 98.3* | 98.3# | 99.2# | 100.6# | 98.9† | 96.4# |
| Total Impurity [%] | 0.74* | 0.96# | 1.2# | 1.3# | 1.3† | 1.4# |
| * one vial<br># average of two vials<br>† average of three vials<br>- means value not determined | | | | | | |

**Embodiments**

1. An anti-fungal composition comprising

    a) caspofungin, or a pharmaceutically acceptable salt thereof, and
    b) at least one bulking agent, wherein the ratio between the amount of caspofungin, or a pharmaceutically acceptable salt thereof, and the amount of bulking agent(s) is at least about 1 : 2 (w/w), and wherein the anti-fungal composition does not comprise a buffering agent.

2. The anti-fungal composition according to embodiment 1, wherein the pharmaceutically acceptable salt of caspofungin is a caspofungin salt of acetic acid, citric acid, tartaric acid, propionic acid, succinic acid, oxalic acid, malic acid, maleic acid, lactic acid, or glutamic acid, preferably a caspofungin salt of acetic acid, more preferably caspofungin acetate or caspofungin diacetate.

3. The anti-fungal composition according to embodiment 1 or 2, wherein the at least one bulking agent is one bulking agent, or wherein the at least one bulking agent comprises two or more bulking agents.

4. The anti-fungal composition according to any one of embodiments 1 to 3, wherein the at least one bulking agent is selected from the group consisting of a monosaccharide, a sugar alcohol, a disaccharide, and a polysaccharide, preferably wherein the at least one bulking agent comprises a disaccharide or a polysaccharide, more preferably wherein the at least one bulking agent comprises a disaccharide, most preferably wherein the at least one bulking agent comprises sucrose.

5. The anti-fungal composition according to any one of embodiments 1 to 4, wherein the ratio between the amount of caspofungin, or a pharmaceutically acceptable salt thereof, and the amount of bulking agent(s) is at least 1 : 5 (w/w), preferably at least 1 : 7 (w/w), more preferably at least 1 : 8 (w/w), more preferably at least 1 : 9 (w/w), more preferably at least 1 : 10 (w/w), more preferably at least 1 : 11 (w/w), more preferably at least 1 : 12 (w/w), more preferably at least 1 : 13 (w/w), most preferably at least 1 : 14 (w/w).

6. The anti-fungal composition according to any one of embodiments 1 to 4, wherein the ratio between the amount of caspofungin, or a pharmaceutically acceptable salt thereof, and the amount of bulking agent(s) is in the range of from about 1 : 5 (w/w) to about 1 : 40 (w/w), preferably in the range of from about 1 : 7 (w/w) to about 1 : 35 (w/w), more preferably in the range of from about 1 : 8 (w/w) to about 1 : 33 (w/w), more preferably in the range of from about 1 : 9 (w/w) to about 1 : 30 (w/w), more preferably in the range of from about 1 : 10 (w/w) to about 1 : 28 (w/w), more preferably in the range of from about 1 : 11 (w/w) to about 1 : 26 (w/w), more preferably in the range of from about 1 : 12 (w/w) to about 1 : 25 (w/w), more preferably in the range of from about 1 : 13 (w/w) to about 1 : 24 (w/w), more preferably in the range of from about 1 : 14 (w/w) to about 1 : 23 (w/w), most preferably in the range of from about 1 : 14 (w/w) to about 1 : 22 (w/w).

7. The anti-fungal composition according to any one of embodiments 1 to 6, wherein the anti-fungal composition is in the form of a lyophilized composition, preferably wherein said lyophilized composition is suitable for reconstitution to form a liquid composition for parenteral administration.

8. The anti-fungal composition according to any one of embodiments 1 to 7, wherein the anti-fungal composition has a density of about 0.5 g/mL or less, preferably of about 0.3 g/mL or less, most preferably the anti-fungal composition has a density in the range of from about 0.1 g/mL to about 0.2 g/mL.

9. The anti-fungal composition according to any one of embodiments 1 to 8, wherein the anti-fungal composition has a water content of about 2 wt.-% or less, preferably about 1.5 wt.-% or less, most preferably about 1 wt.-% or less, based on the total weight of the anti-fungal composition.

10. The anti-fungal composition according to any one of embodiments 1 to 9, wherein the anti-fungal composition comprises caspofungin or its pharmaceutically acceptable salt in an amount of from about 8 $\mu$mol to about 125 $\mu$mol, preferably in an amount of from about 20 $\mu$mol to about 85 $\mu$mol, most preferably in an amount of from about 40 $\mu$mol to about 65 $\mu$mol.

11. The anti-fungal composition according to any one of embodiments 1 to 10, wherein the anti-fungal composition has a shelf storage of at least 24 months.

12. The anti-fungal composition according to any one of embodiments 1 to 10, wherein the anti-fungal composition comprises at least 95 % (n/n), preferably at least 96 % (n/n), more preferably at least 97 % (n/n), most preferably at least 98 % (n/n) of caspofungin or its pharmaceutically acceptable salt after storage for 24 months at 25 °C and 60 % relative humidity based on the amount of caspofungin or pharmaceutically acceptable salt thereof before storage.

13. The anti-fungal composition according to any one of embodiments 1 to 12, wherein the anti-fungal composition comprises 5 % or less of impurities, preferably 4 % or less of impurities, more preferably 3 % or less of impurities, most preferably 2 % of impurities.

14. The anti-fungal composition according to any one of embodiments 1 to 13, wherein the anti-fungal composition comprises 5 % or less of impurities, preferably 4 % or less of impurities, more preferably 3 % or less of impurities, most preferably 2 % of impurities after storage for 8 months at 40 °C.

15. A method of manufacturing an anti-fungal composition according to any one of embodiments 1 to 14, comprising the steps of

a) providing a first solution of at least one bulking agent in water;
b) dissolving caspofungin or its pharmaceutically acceptable salt in said first solution;
c) freezing the solution obtained in step b) at a temperature of from about -60 °C to about -40 °C for about 3 h to about 10 h to provide a frozen solution; and
d) freeze drying said frozen solution to provide the anti-fungal composition.

16. The method according to embodiment 15, wherein the first solution comprises the at least one bulking agent in an amount of from about 2 wt.-% to about 25 wt.-%, preferably of from about 5 wt.-% to about 20 wt.-%, more preferably of from about 7 wt.-% to about 15 wt.-%, more preferably of from about 8 wt.-% to about 12 wt.-%, more preferably of from about 9 wt.-% to about 11 wt.-%, most preferably in an amount of about 10 wt.-% based on the total weight of the first solution.

17. The method according to embodiment 15 or 16, wherein freeze drying is carried out for about 96 h or less, preferably for about 72 h or less.

18. An anti-fungal composition prepared by the method according to any one of embodiments 15 to 17.

19. A sealed container comprising the anti-fungal composition according to any one of embodiments 1 to 14 or 18, preferably wherein the sealed container is a glass vial or a plastic vial.

20. The anti-fungal composition according to any one of embodiments 1 to 14 or 18 for use in the treatment or prevention of a fungal infection.

**Claims**

1. An anti-fungal composition comprising

a) caspofungin, or a pharmaceutically acceptable salt thereof, and
b) at least one bulking agent,

wherein the ratio between the amount of caspofungin, or a pharmaceutically acceptable salt thereof, and the amount of bulking agent(s) is at least about 1 : 2 (w/w), and wherein the anti-fungal composition does not comprise a buffering agent.

2. The anti-fungal composition according to claim 1, wherein the pharmaceutically acceptable salt of caspofungin is a caspofungin salt of acetic acid, citric acid, tartaric acid, propionic acid, succinic acid, oxalic acid, malic acid, maleic acid, lactic acid, or glutamic acid, preferably a caspofungin salt of acetic acid, more preferably caspofungin acetate or caspofungin diacetate.

3. The anti-fungal composition according to claim 1 or 2, wherein the at least one bulking agent is one bulking agent, or wherein the at least one bulking agent comprises two or more bulking agents.

4. The anti-fungal composition according to any one of claims 1 to 3, wherein the at least one bulking agent is selected from the group consisting of a monosaccharide, a sugar alcohol, a disaccharide, and a polysaccharide, preferably wherein the at least one bulking agent comprises a disaccharide or a polysaccharide, more preferably wherein the at least one bulking agent comprises a disaccharide, most preferably wherein the at least one bulking agent comprises sucrose.

5. The anti-fungal composition according to any one of claims 1 to 4, wherein

a) the ratio between the amount of caspofungin, or a pharmaceutically acceptable salt thereof, and the amount of bulking agent(s) is at least 1 : 5 (w/w), preferably at least 1 : 7 (w/w), more preferably at least 1 : 8 (w/w), more preferably at least 1 : 9 (w/w), more preferably at least 1 : 10 (w/w), more preferably at least 1 : 11 (w/w), more preferably at least 1 : 12 (w/w), more preferably at least 1 : 13 (w/w), most preferably at least 1 : 14 (w/w), or
b) the ratio between the amount of caspofungin, or a pharmaceutically acceptable salt thereof, and the amount of bulking agent(s) is in the range of from about 1 : 5 (w/w) to about 1 : 40 (w/w), preferably in the range of from about 1 : 7 (w/w) to about 1 : 35 (w/w), more preferably in the range of from about 1 : 8 (w/w) to about 1 : 33 (w/w), more preferably in the range of from about 1 : 9 (w/w) to about 1 : 30 (w/w), more preferably in the range of from about 1 : 10 (w/w) to about 1 : 28 (w/w), more preferably in the range of from about 1 : 11 (w/w) to about 1 : 26 (w/w), more preferably in the range of from about 1 : 12 (w/w) to about 1 : 25 (w/w), more preferably in the range of from about 1 : 13 (w/w) to about 1 : 24 (w/w), more preferably in the range of from about 1 : 14 (w/w) to about 1 : 23 (w/w), most preferably in the range of from about 1 : 14 (w/w) to about 1 : 22 (w/w).

6. The anti-fungal composition according to any one of claims 1 to 5, wherein the anti-fungal composition is in the form of a lyophilized composition, preferably wherein said lyophilized composition is suitable for reconstitution to form a liquid composition for parenteral administration; and/or
wherein the anti-fungal composition has a density of about 0.5 g/mL or less, preferably of about 0.3 g/mL or less, most preferably the anti-fungal composition has a density in the range of from about 0.1 g/mL to about 0.2 g/mL.

7. The anti-fungal composition according to any one of claims 1 to 6, wherein the anti-fungal composition has a water content of about 2 wt.-% or less, preferably about 1.5 wt.-% or less, most preferably about 1 wt.-% or less, based on the total weight of the anti-fungal composition.

8. The anti-fungal composition according to any one of claims 1 to 7, wherein

a) the anti-fungal composition has a shelf storage of at least 24 months; and/or
b) the anti-fungal composition comprises at least 95 % (n/n), preferably at least 96 % (n/n), more preferably at least 97 % (n/n), most preferably at least 98 % (n/n) of caspofungin or its pharmaceutically acceptable salt after storage for 24 months at 25 °C and 60 % relative humidity based on the amount of caspofungin or pharmaceutically acceptable salt thereof before storage.

9. The anti-fungal composition according to any one of claims 1 to 8, wherein

a) the anti-fungal composition comprises 5 % or less of impurities, preferably 4 % or less of impurities, more preferably 3 % or less of impurities, most preferably 2 % of impurities; and/or.
b) the anti-fungal composition comprises 5 % or less of impurities, preferably 4 % or less of impurities, more preferably 3 % or less of impurities, most preferably 2 % of impurities after storage for 8 months at 40 °C.

10. A method of manufacturing an anti-fungal composition according to any one of claims 1 to 9, comprising the steps of

a) providing a first solution of at least one bulking agent in water;
b) dissolving caspofungin or its pharmaceutically acceptable salt in said first solution;
c) freezing the solution obtained in step b) at a temperature of from about -60 °C to about -40 °C for about 3 h to about 10 h to provide a frozen solution; and
d) freeze drying said frozen solution to provide the anti-fungal composition.

11. The method according to claim 10, wherein the first solution comprises the at least one bulking agent in an amount of from about 2 wt.-% to about 25 wt.-%, preferably of from about 5 wt.-% to about 20 wt.-%, more preferably of from about 7 wt.-% to about 15 wt.-%, more preferably of from about 8 wt.-% to about 12 wt.-%, more preferably of from about 9 wt.-% to about 11 wt.-%, most preferably in an amount of about 10 wt.-% based on the total weight of the first solution.

12. The method according to claim 10 or 11, wherein freeze drying is carried out for about 96 h or less, preferably for about 72 h or less.

13. An anti-fungal composition prepared by the method according to any one of claims 10 to 12.

14. A sealed container comprising the anti-fungal composition according to any one of claims 1 to 9 or 13, preferably wherein the sealed container is a glass vial or a plastic vial.

15. The anti-fungal composition according to any one of claims 1 to 9 or 13 for use in the treatment or prevention of a fungal infection.

Europäisches
Patentamt

European
Patent Office

Office européen
des brevets

# EUROPEAN SEARCH REPORT

**Application Number**

EP 22 20 5503

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2015/072923 A1 (SHIRODE SWAPNIL P [IN] ET AL) 12 March 2015 (2015-03-12) * the claims; paragraphs 4-19, 21-44, and the examples * ----- | 1-15 | INV. A01N25/10 A01N37/18 A61K38/12 A01P3/00 |
| Y | US 2010/137197 A1 (MITTAL SACHIN [US] ET AL) 3 June 2010 (2010-06-03) * the claims; paragraphs 3-52, 56, 66-69, 78-86, 94, and the examples * ----- | 1-15 | |
| Y | US 2013/028940 A1 (WELZ CHRISTIAN [AT] ET AL) 31 January 2013 (2013-01-31) * the claims; paragraphs 127, 158, 161, 176-189; examples 2-4, 6, 8-12, 16-18 * ----- | 1-15 | |
| X | US 2014/221274 A1 (HONG YUNHAI [CN] ET AL) 7 August 2014 (2014-08-07) | 1-15 | |
| Y | * the claims; paragraphs 2-4, 16-88, 94-96, 100-106; comparative examples 1 and 2 and examples 1 and 2 * ----- | 1-15 | |
| Y | CN 101 516 387 B (SANDOZ AG) 4 June 2014 (2014-06-04) * the whole document * ----- | 1-15 | **TECHNICAL FIELDS SEARCHED (IPC)** A01N A61K |
| X | CN 102 614 493 B (SHANGHAI TECHWELL BIOPHARM CO) 11 December 2013 (2013-12-11) | 1-15 | |
| Y | * the whole document; in particular, embodiments 1-3 * ----- | 1-15 | |

The present search report has been drawn up for all claims

| Place of search | Date of completion of the search | Examiner |
|---|---|---|
| Munich | 6 April 2023 | Lorenzo Varela, M |

EPO FORM 1503 03.82 (P04C01)

## ANNEX TO THE EUROPEAN SEARCH REPORT
### ON EUROPEAN PATENT APPLICATION NO.

EP 22 20 5503

This annex lists the patent family members relating to the patent documents cited in the above-mentioned European search report.
The members are as contained in the European Patent Office EDP file on
The European Patent Office is in no way liable for these particulars which are merely given for the purpose of information.

06-04-2023

| Patent document cited in search report | Publication date | Patent family member(s) | | Publication date |
|---|---|---|---|---|
| US 2015072923 A1 | 12-03-2015 | AU | 2011304408 A1 | 28-03-2013 |
| | | CA | 2810112 A1 | 29-03-2012 |
| | | CN | 103118663 A | 22-05-2013 |
| | | DK | 2618814 T3 | 10-10-2016 |
| | | EP | 2618814 A1 | 31-07-2013 |
| | | ES | 2593727 T3 | 12-12-2016 |
| | | HR | P20161236 T1 | 18-11-2016 |
| | | HU | E030464 T2 | 29-05-2017 |
| | | JP | 5914486 B2 | 11-05-2016 |
| | | JP | 2013537212 A | 30-09-2013 |
| | | KR | 20130136466 A | 12-12-2013 |
| | | PL | 2618814 T3 | 31-01-2017 |
| | | PT | 2618814 T | 06-10-2016 |
| | | RU | 2013114993 A | 27-10-2014 |
| | | UA | 111599 C2 | 25-05-2016 |
| | | US | 2012101030 A1 | 26-04-2012 |
| | | US | 2015072923 A1 | 12-03-2015 |
| | | WO | 2012038371 A1 | 29-03-2012 |
| | | ZA | 201302113 B | 28-05-2014 |
| US 2010137197 A1 | 03-06-2010 | AU | 2008269140 A1 | 31-12-2008 |
| | | CA | 2692053 A1 | 31-12-2008 |
| | | EP | 2170362 A1 | 07-04-2010 |
| | | JP | 5537425 B2 | 02-07-2014 |
| | | JP | 2010531355 A | 24-09-2010 |
| | | US | 2010137197 A1 | 03-06-2010 |
| | | US | 2013023462 A1 | 24-01-2013 |
| | | WO | 2009002481 A1 | 31-12-2008 |
| US 2013028940 A1 | 31-01-2013 | BR | PI0715113 A2 | 04-06-2013 |
| | | CA | 2657817 A1 | 31-01-2008 |
| | | EP | 2049142 A1 | 22-04-2009 |
| | | EP | 2340846 A1 | 06-07-2011 |
| | | ES | 2401299 T3 | 18-04-2013 |
| | | HR | P20130109 T1 | 31-03-2013 |
| | | JP | 5373606 B2 | 18-12-2013 |
| | | JP | 2009544662 A | 17-12-2009 |
| | | KR | 20090046865 A | 11-05-2009 |
| | | PL | 2049142 T3 | 28-06-2013 |
| | | SI | 2049142 T1 | 28-02-2013 |
| | | US | 2009170753 A1 | 02-07-2009 |
| | | US | 2013028940 A1 | 31-01-2013 |
| | | WO | 2008012310 A1 | 31-01-2008 |
| US 2014221274 A1 | 07-08-2014 | CN | 102488889 A | 13-06-2012 |
| | | CN | 103386117 A | 13-11-2013 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

**EP 22 20 5503**

This annex lists the patent family members relating to the patent documents cited in the above-mentioned European search report.
The members are as contained in the European Patent Office EDP file on
The European Patent Office is in no way liable for these particulars which are merely given for the purpose of information.

**06-04-2023**

| Patent document cited in search report | Publication date | Patent family member(s) | Publication date |
|---|---|---|---|
| | | DE 112012003996 T5 | 14-08-2014 |
| | | ES 2519240 A2 | 06-11-2014 |
| | | KR 20140069272 A | 09-06-2014 |
| | | US 2014221274 A1 | 07-08-2014 |
| | | WO 2013044788 A1 | 04-04-2013 |
| CN 101516387 B | 04-06-2014 | NONE | |
| CN 102614493 B | 11-12-2013 | NONE | |

EPO FORM P0459

For more details about this annex : see Official Journal of the European Patent Office, No. 12/82

**page 2 of 2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *CHEMICAL ABSTRACTS,* 162808-62-0

- *CHEMICAL ABSTRACTS,* 179463-17-3